# EUROPEAN PATENT APPLICATION

(11) **EP 4 273 250 A1**
(43) Date of publication of application: **08.11.2023**
(21) Application number: 21915743.5
(22) Date of filing: 28.12.2021
(51) Int. Cl.: C12N 15/70, C12N 9/10, C12P 19/18

(54) **RECOMBINANT MICROORGANISM EXPRESSING FUCOSYLTRANSFERASE, AND METHOD OF PRODUCING 2'-FUCOSYLLACTOSE USING SAME**

(30) Priority: 31.12.2020 KR 20200189490
(71) Applicant: Samyang Corporation, Jongno-gu Seoul 03129 (KR)
(72) Inventor: HONG, Eun-Young, Seoul 07340 (KR); PARK, Bu-Soo, Hanam-si, Gyeonggi-do 13014 (KR); LEE, Sanghee, Gwangju-si, Gyeonggi-do 12788 (KR); CHOI, Eunsoo, Seongnam-si, Gyeonggi-do 13588 (KR)
(74) Representative: Schrell, Andreas
(86) International application number: PCT/KR2021/020001
(87) International publication number: WO 2022/145945

(57) **Abstract**

The present invention relates to a microorganism introduced with 2'-fucosyltransferase and a method for producing 2'-fucosyllactose using the same, and the fucosyltransferase derived from the microorganism of the present invention is safe and has high activity compared to conventional enzymes derived from *Helicobacter pylori.*

## Description

### [TECHNICAL FIELD]

The present invention relates to a recombinant microorganism transformed to express fucosyltransferase and a method for producing 2'-fucosyllactose using the same.

### [BACKGROUND ART]

137 kinds including 3 kinds of oligosaccharides with the highest contents among human milk are fucosylated and ratio thereof is about 77%, and the rest oligosaccharides are mostly sialylated (39 kinds) and correspond to about 28%. Among them, in particular, 2'-fucosyllactose and 3'-fucosyllactose have a prebiotic effect, an effect of inhibiting intestinal attachment of pathogens, and an effect of regulating an immunoregulatory system, and the like. On the prebiotic effect, the most research has been conducted so far, and human milk oligosaccharides of breast milk selectively enhance growth and development of *Bifidobacterium bifidum,* which grows as a dominant species initially in intestines of infants, whereas they cannot be used by harmful bacteria. In addition, the human milk oligosaccharides are known to inhibit intestinal attachment of pathogens, and this is because structures of cell wall polysaccharides of a pathogen that binds to intestinal lectin is often similar to some structures of human milk oligosaccharides. Therefore, it seems that breast-feeding infants have resistance to infection by pathogens as human milk oligosaccharides provide water-soluble ligand analogues. However, it is known that due to mutation of fucosyltransferase which synthesizes 2'-fucosyllactose, about 20% of women cannot synthesize it in the body properly. Due to this, industrial production of 2'-fucosyllactose is required.

Some of human milk oligosaccharides are found also in milk of most mammals (primates, cattle, pigs, goats, sheep, elephants) in a small amount. Among them, the milk of goats is most similar to the composition of the human milk oligosaccharides, and due to this, a large-scale membrane use separation technology from whey by-products of a goat milk cheese production process has been suggested. In addition, when recently developed solid-phase synthesis is used, Lewis X-Lewis Y saccharides can be produced within one day, and when a crystalline intermediate technology is used, rapid synthesis of 2'-fucosyllactose becomes possible. However, despite development of the above separation method or a chemical synthesis method, several obstacles remain for mass production for industrialization of human milk oligosaccharides, as the present technologies are not suitable for a food or pharmaceutical industry due to low stereoselectivity, low total yield and use of toxic reagents, and the like. Thus, more environmentally friendly and high yield producing method is required, and recently, a method for producing a high-concentration 2'-FL by a microorganism has been reported. However, a 2'-fucosyllactose synthesis gene uses 2-fucosyllactose secured from a Bio Safety Level 2 strain, *Helicobacter pylori,* and thus, it may act as a negative factor in perception of consumers when applied mainly to powdered milk for replacing breast-feeding and food for infants, and it is not appropriate for domestic production and sales permission. Therefore, it is needed to produce 2'-fucosyllactose through development of a Bio Safety Level 1 strain by applying a gene with higher stability as a food additive.

### [DISCLOSURE]

### [TECHNICAL PROBLEM]

The present invention is to provide a recombinant microorganism in which 2'-fucosyltransferase derived from a Biosafety level 1 strain, for example, *Bacillus megaterium* or *Akkermansia muciniphila* is inserted, as a microorganism having transferase producing fucosyllactose which is material for a food, cosmetic and pharmaceutical, and is to provide an effective method for producing 2'-fucosyllactose using the same.

### [TECHNICAL SOLUTION]

An embodiment of the present invention relates to a recombinant microorganism, transformed to express α-1,2-fucosyltransferase. The α-1,2-fucosyltransferase may comprise at least one selected from the group consisting of SEQ ID NOs: 1 to 5.

In the present invention, a polypeptide having 2-fucose transferase activity is not limited to the amino acid sequences of SEQ ID NOs: 1 to 5, and includes amino acid sequences formed by substitution, insertion or deletion of some amino acid residues in the amino acid sequences of SEQ ID NOs: 1 to 5, and when a protein or polypeptide modified with these amino acids has 2-fucose transferase activity, it may convert lactose and GDP-fucose as substrates into fucosyllactose.

For example, a nucleic acid sequence encoding the α-1,2-fucosyltransferase may comprise at least one nucleic acid sequence selected from the group consisting of SEQ ID NOs: 14 to 18.

The recombinant microorganism may be a GRAS (Generally Recognized As Safe) microorganism. Stability of a gene used for producing 2'-fucosyllactose is not good, as a gene obtained from *Helicobacter pylori* has been used so far. In order to solve this problem, the recombinant microorganism according to the present invention may be a Biosafety Level 1 microorganism. As fucosyltransferase derived from *Helicobacter pylori* is Biosafety Level 2, it is not suitable for an enzyme for producing food materials. However, the fucosyltransferase according to the present invention is Biosafety level 1, so it has an advantage that there is no safety concern raised by consumers.

The recombinant microorganism according to the present invention can enhance stability and productivity, as the 2'-fucosyllactose productivity is significantly increased, compared to a control group comprising α-1,2-fucosyltransferase derived from *Helicobacter pylori.*

Specifically, the recombinant microorganism according to one embodiment of the present invention may have 2'-fucosyllactose productivity of 2 times or more, 2.1 times or more, 2.5 times or more, 3 times or more, 3.5 times or more, 4 times or more, 4.5 times or more, 5 times or more, 5.5 times or more, 6 times or more, 6.5 times or more, 7 times or more, or 7.5 times or more, compared to the control group comprising α-1,2-fucosyltransferase derived from *Helicobacter pylori.* Therefore, the recombinant microorganism may be usefully used in preparation of α-1,2-fucose, and may enhance the production yield of α-1,2-fucose.

The recombinant microorganism according to one embodiment of the present invention may not have 2'-fucosyllactose productivity before recombination, and may obtain 2'-fucosyllactose productivity by recombination.

For the method for transforming the recombinant microorganism, all transformation methods known in the art may be selected and used without limitation, and for example, it may be selected from fusion of bacterial protoplasts, electroporation, projectable bombardment, and infection using a virus vector, and the like.

The recombinant microorganism according to the present invention may be *Bacillus* sp. microorganism, *Corynebacterium* sp. microorganism, *Escherichia* sp. microorganism, or yeast.

Specifically, the *Bacillus* sp. microorganism may be *Bacillus megaterium, Bacillus subtilis, Bacillus cereus, Bacillus coagulans, Bacillus licheniformis,* or *Bacillus stearothermophilus.*

Specifically, the *Corynebacterium* sp. microorganism may be *Corynebacterium glutamicum.*

Specifically, the *Escherichia* sp. microorganism may be *Escherichia coli.*

Specifically, the yeast may be *Saccharomyces cerevisiae,* or *Candida utilis.*

The recombinant microorganism according to one embodiment of the present invention may have fucose synthesis activity, or improve fucose synthesis activity. In order to produce 2'-fucosyllactose, it is same that any microorganism fundamentally need to be introduced foreign α-1,2-fucosyltransferase, but genetic characteristics of the strain of each microorganism itself are different, so *Escherichia coli* or *Bacillus megaterium* can produce 2'-fucosyllactose by inserting only α-1,2-fucosyltransferase, but *Bacillus subtilis, Corynebacterium,* yeast microorganism, and the like may additionally be introduced fucose synthase.

Specifically, the recombinant microorganism according to one embodiment of the present invention may be characterized by retaining or overexpressing at least one selected from the group consisting of GDP-D-mannose-4,6-dehydratase, GDP-L-fucose synthase (WcaG), phosphomannomutase, and GTP-mannose-1-phosphate guanylyltransferase. Accordingly, the recombinant microorganism according to one embodiment of the present invention can further enhance the α-1,2-fucose production yield by improving fucose synthesis activity, in addition to the characteristic of expressing α-1,2-fucosyltransferase.

For example, the fucose synthase may comprise at least one amino acid sequence selected from the group consisting of SEQ ID NOs: 19 to 22.

For example, the GTP-mannose-1-phosphate guanylyltransferase may comprise the amino acid sequence of SEQ ID NO: 19, or be encoded by the nucleic acid sequence of SEQ ID NO: 23.

For example, the phosphomannomutase may comprise the amino acid sequence of SEQ ID NO: 20, or be encoded by the nucleic acid sequence of SEQ ID NO: 24.

For example, the GDP-L-fucose synthase (WcaG) may comprise the amino acid sequence of SEQ ID NO: 21, or be encoded by the nucleic acid sequence of SEQ ID NO: 25.

For example, the GDP-D-mannose-4,6-dehydratase may comprise the amino acid sequence of SEQ ID NO: 22, or be encoded by the nucleic acid sequence of SEQ ID NO: 26.

The recombinant microorganism according to one embodiment of the present invention may express or overexpress a lactose membrane transport protein. The lactose membrane transport protein may improve 2'-fucosyllactose productivity, by transporting lactose as a substrate of 2'-fucosyllactose into a cell.

Specifically, the lactose membrane transport protein may be Lac12 and/or LacY.

For example, the Lac12 may have an amino acid sequence of SEQ ID NO: 27. For example, a nucleic acid sequence encoding the Lac12 may comprise a nucleic acid sequence of SEQ ID NO: 29. As an example, the Lac12 may be encoded by the nucleic acid sequence of SEQ ID NO: 29.

For example, the LacY may have the amino acid sequence of SEQ ID NO: 28. For example, a nucleic acid sequence encoding the LacY may comprise a nucleic acid sequence of SEQ ID NO: 30. As an example, the LacY may be encoded by the nucleic acid sequence of SEQ ID NO: 30.

Other one embodiment of the present invention relates to a composition for producing 2'-fucosyllactose, comprising at least one selected from the group consisting of α-1,2-fucosyltransferase obtained using the recombinant microorganism according to the present invention, a microbial cell of the microorganism, a culture of the microorganism, a lysate of the microorganism, and an extract of the lysate or culture. The recombinant microorganism, the α-1,2-fucosyltransferase, the 2'-fucosyllactose, and the like are as described above.

The culture comprises an enzyme produced from the recombinant microorganism, and it may comprise the recombinant microorganism, or may be in a cell-free form not comprising the recombinant microorganism. The lysate means a lysate obtained by lysing the recombinant microorganism or a supernatant obtained by centrifuging the lysate, and comprises an enzyme produced from the recombinant microorganism. In the present description, unless otherwise mentioned, the recombinant microorganism used in preparation of 2'-fucosyllactose is used to mean at least one selected from the group consisting of a microbial cell of the microorganism, a culture of the microorganism and a lysate of the microorganism.

Other one embodiment of the present invention relates to a method for producing 2'-fucosyllactose, comprising a step of culturing the recombinant microorganism according to the present invention in a medium comprising lactose.

The method for producing may be producing 2'-fucosyllactose, by reacting at least one selected from the group consisting of α-1,2-fucosyltransferase obtained from a culture, a microbial cell of the microorganism, a culture of the microorganism, a lysate of the microorganism, and an extract of the lysate or culture, with a lactose-containing raw material.

### [ADVANTAGEOUS EFFECTS]

The present invention can provide a recombinant microorganism without concern of consumers in terms of stability as fucosyltransferase derived from a biosafety level 1 strain is introduced, and a method for producing 2'-fucosyllactose using the same.

### [BRIEF DESCRIPTION OF THE DRAWINGS]

FIG. 1 is a drawing which shows SDS-PAGE result for the protein expression and expected protein size of the selected enzymes according to the Example of the present invention.
FIG. 2 is a drawing which shows the 2FL production tendency of the flask reaction (top) and test tube reaction (bottom) according to the Example of the present invention.
FIG. 3 is a drawing which shows the result of comparing the 2FL production activity using *E.coli* as a host for α-1,2-fucosyltransferase according to one embodiment of the present invention.
FIG. 4 is a drawing which shows the result of comparing various 2FL productivity using *B. megaterium* 14581 as a host.
FIG. 5 is a drawing which shows the GDP-fucose cassette according to the Example of the present invention.
FIG. 6 is a drawing which confirms the 2FL productivity using the strain introduced by GDP-fucose cassette according to the Example of the present invention.

### [MODE FOR INVENTION]

Hereinafter, the present invention will be described in more detail by the following Examples. However, these Examples are intended to illustrate the present invention only, and the scope of the present invention is not limited by these Examples.

### Example 1. Screening of a-1,2-fucosyltransferase using bioinformatics tools

By using bioinformatics tools, candidates of a-1,2-fucosyltransferase enzymes (hereinafter, 2FT) derived from Biosafety level 1 microorganism were selected. Specifically, the enzymes having>90% homology sequence based on the bacteria origin and GRAS bacteria origin were removed among enzymes expected to have a-1,2-fucosyltransferase function in National Center for Biotechnology Information (NCBI)(https://www.ncbi.nlm.nih.gov/protein/), and the presence or absence of a membrane protein was analyzed using a protein morphology visualization program (http://wlab.ethz.ch/protter).

By using computer bioinformatics tools, 32 candidate groups were selected (Table 1). In addition, when presence or absence of a membrane protein that becomes problematic for protein expression was predicted, the remaining 31 enzymes except for the *Lactococcus lactis* origin among the 32 candidate groups, were not predicted to have apparent membrane protein.

**[Table 1]**

| Selection criteria | Candidate group |
|---|---|
| Bacteria | 6431 |
| GRAS bacteria (ex) *Acetobacter, Bacillus, Lactobacillus* etc) | 67 |
| Duplication exclusion (exclusion with >90% homology sequence, exclusion of partial sequence) | 32 |

Biosafety level 1 Bm2FT was subsequently confirmed to have the reactivity among the candidate groups, and thus, additional candidate groups were searched for *Bacillus megaterium*derived enzymes, based on the Bm2FT protein sequence homology. The enzymes were searched using BLAST program (https://blast.ncbi.nlm.nih.gov/Blast.cgi) based on the sequence information of Bm2FT. A list of the selected enzyme candidates is shown in Table 2.

**[Table 2]**

| Name | Sequence | SEQ ID NO: |
|---|---|---|
| Am2FT_2 | MNMERKTGLMNKKYVSPCFLPGMRLGNIMFTLAAACAHARTVGV ECRVPWAYNDASLMLRSRLGGWVLPSTPCGTNEPPSWQEPSFAYC PVPSRIRTGGLRGYFQSARYFEGQEAFIRALFAPLTAEKEPGAVGIHI RLGDYRRLRDKHRILDPGFLRRAAGHLSSGKNRLVLFSDEPDEAAE MLARVPAFGRFALEIDRGAPCESLRRMTAMEELVMSCSSFSWWGA WLGNTRKVIVPRDWFVGGVEDYRDIYLPHWVTL | 1 |
| Bm2FT | MKIVQISSGLGNQLFQYALYKRLSMNNNDVFLDVETSYQLNKNQH NGYEIERIFSIQPSHATKGMIDELADVDNRLINRLRRKLFGPKNSMY TETKEFSYDCEVFTKDGIYIKGYWQNYNYFKEIEDDLKNELVFKKA LDLKNSHLINQMNKEISVSIHVRRGDYYLNKEYENKFGNIADLDYY LKAINFIKKEVDDPKFYVFSDDIKWAKENLNLTDNVTYVEHNKGS DSYKDMRLMTCCKHNIIANSTFSWWGAFLNENKNKIVIAPGKWIN VEGVGGINLFPEGWIVY | 2 |
| Bm2FT_2 | MGCIKRLFLYEYGGRCFLVIVKIKGGFGNQLFTYASAYAISQELQQ NLIMDKVIYDLDYFRKFELPSLSLKYDQMLISKFVPNTKVKTLIYKV LRRLKLKGFTEVHEKKEFSFDENIYNLSGDIYLDGYWQNYRYFHK YYKDLSEMFVPRETPRKEVTDYITSLRGVNSVAMHVRRGDYKTFN GGKCLSLDYYIKAMEYFNSNDVQFYVFTDDIDFCEKNLPNSENINY VSRSEKLTDIEEFFIMKECKNFIIANSSFSWWAAYLSEQKADSLIVAP VVDMWKRDFYPDEWVALNTHLE | 3 |
| Bm2FT_3 | MIIVQIKGGLGNQLFSYASAYGIARENETELIIDRYIYDTSYSLRKYM LDFFPEIDEALLLKYIPKKNKISQIMYKLIRKSKLKYKYKAQLFLEEE EFKFTRISTQNENLYLNGYWQSYVYFDKYRNDIIKKFTPLVSFNQD GNQLLNEIKSYNSVAIHVRRGDYINFKGGKCLDSSYYIKAMKHLYN LKGKNLFFYIFTDDVEYCKRIFKNVANVKFIGEEAKLSDFEEFTLMT HCKNLIIGNSSFSWWAAYLASCKDKAVIAPVVDMWTEDFYLPEWI KIKADLQ | 4 |
| Bs2FT | MKIVHISSGLGNQMFQYALYKKLSLIQDNVFLDTITSYQLYPNQHN GYELEKVFTIKPRHASKELTYNLSDLDNSVTSRIRRKLIGSKKSMYI EHKEFEYDPNLFYQENIYIKGYWQNYDYFKDIENELKNDFTFQRAL DEKNNNLAIKINNENSISIHVRRGDYYLNRKNQEKFGDIANLEYYS KAISYIKERIDNPKFYIFSDNVEWVKQNLNSLEEAVYIDYNVGNDSY KDMQLMSLCKHNIIANSSFSWWGAFLNKNMEKIVIAPGKWINMKG VKKVNLFPKDWIIY | 5 |
| Bm2FT_4 | MIIVRVIGGLGNQMFQYALYKSLENEGKEVKLDLTGFGDYDLHNG YELNKIFNINENVATKDEINKLIKLPDNKVLSMIKRKFFSSTINYYSQ DQFKYLSEIFQLDNVYLDGYWQSEKYFQGIKEVIRKEFKFKGEPNP KNIEMVKLMRGSNSVSIHFRRGDYISNPDAYKVHGGITTIHYYENA | 6 |
| | VKEIKSKVKEPKFFIFSDDIKWVKENFKLEDAFFIDWNTGSESYRDI ELMSNCKHNIIANSTFSWWGAWLNKNKNKIVIAPNQWFNTIDTED VIPDTWQCINTF | |
| Bt2FT | MNEIHVLLTGRLGNQLFQYAFARSLQKQYGGRIVCNTFDLDHRSE KSKVADQKFSYAMGDFKLDENVALEDAALPWYADFSSPLIKPVKK LFPRRYFDFMARRGYLMWQRTDYMPIPKLECEDVFAWGWWQDIR YFQDVQTELSDEVVPVTDPLPENQYIYNAASGEESVCISIRCGNYFN PTVKKLLYVCTPEYFRNAVESITKRLTHPKFIVFTDDVDWVKENIRF ESAYPQYEFLYERGSDTVEEKIRMMTLCKHFIISNSTFSWWAQFLSK SENKIVIAPDRWFVDGRRIGLYMHGWTLIPAGQ | 7 |
| Fp2FT | MIYVEMHGRLGNQMFQYAAARALQEKNNQPIMLSFRKVIGANTE GTAGWENSLKYFNVKPCEYYMGKKSLVTEYPVEYRLLCYAYALS YKPLMNNMNRWYEYQVKCCRFLDRFGIRWIANGYYDFHYNGLKN YLLNGSFESPKYFDSIRDKLLEEFTPREEERKENKRLYEQIRKRNSV CLSVRHFQLTGKQADMYDVCSLEYYQTAIRKMCELIENPLFVVFSD DIEWVKNTIDLSRVEVVYETPGNPVWEKLRLMYSCKNFIIPNSTFA WWAQYLSRNPDKYVLCPAKWFNNNFESPLIASQWVRIDREGNIVN E | 8 |
| Gs2FT | MKIVKVIGGLGNQMFQYAFYRNLKAKFQEVKLDITAFETYKLHNG YELERVFDIKPEYATKKEIYPLTTNRNSKISKIKRRIFGGKETEYIEK DLKFDPEVFKVTGDVYFEGYWQTEKYFKEIEDLIRKDFQFKNPLTN KNLELSNKIKNENSVSIHVRRGDYYTSKKAERKHGNIATIEYYQKA VRKITEFVDNPVFYIFSDDIPWVKENLKLENEVIYVDWNKGLDSYID MQLMSICKHNIIANSTFSWWGAWLNQNKNKIVIAPSRWINNKRLD TSDVIPKEWIKI | 9 |
| Lg2FT | MLYVEMDGRCGNQLFHYAVARYIQLAIGNKEKLCLNFNKIFEKKD ENNGWVDYLKDFKTVPYSYYSKSGTILKNESNFIQKIAIGLKAIQIK SLTKKSRQEQADKAEVGQRTLNKLGVYWVREGVNQIYPYKNNKIL VSGICESNFIYEIQEQLQKELIPVTPVSSLNKSLLEKIDNCNSVCISVR RGDFFNNKNAKKYGVCSPEYYIRAKKYFDKKRLENTVYFCFSDDIE WCKENLKFTDKNVIFVSQEMPVYETLRLMSHCKHFILSNSTFSWW GQFLSEYKDKIVVSPARWNNDGYDTNLIDKNWILIDA | 10 |
| Ll2FT | MIYVEIRGNLGNQLFIYATAKKIQKLTGQKIQLNTTTLNKYFPNYKF GLSEFIMEDPDCFIESYKKLPWFTNEYLLPIKIFKKILNKTPKINKILS DFFFKAFEKKGYFIWRGETFKKFSLGNHKNYYLSGFWQSEDYFYDI RDELLEIITPINSIRECNFELLNLIRNSESICVSIRRGDYVDNPKISAIY NVCDINYFIESVNEIKKNVVNVKVICFSDDVEWVKKNIKFDCETHY ETYGNSLSEKVQLMSSCKHFVLSNSSFSWWTEFLSIRGGITIAPKNW YADEREADIYRKNWIYLEDKTEEE | 11 |
| Ls2FT | MEEIHTLLTGRLGNQLFQYAFARNLQKQYGGQIYCDVYELEHRMS KVADEKFSYAMSGFKLDAGVIREDQAFPWYADFSNPVIKPIKKAM PRKYFELMARRGYLMWQRSDYMPIPVLDTQRVFASGWWQDIRYL QNVQEELSDEIVPITNPLQENRYIYDAAHDRDSVCISIRCGNYFNPT VKKLLYVCNPQYFHDSVKRISQMLAHPKFIVFTDDVSWVKEHLKF EDTYPQFEFLYERGCDTAEEKIRMMAMCNNFIISNSTFSWWAQFLS KNKEKIVIAPDKWFVDGRKIGLYMDGWTLVPAGR | 12 |
| Ri2FT | MIAVKIGDGMGNQLFNYACGYAQARRDGDSLVLDISECDNSTLRD FELDKFHLKYDKKESFPNRNLGQKIYKNLRRALKYHVIKEREVYH | 13 |
| | NRDHRYDVNDIDPRVYKKKGLRNKYLYGYWQHLAYFEDYLNEIT AMMTPAYEQSETVKKLQEEFKKTPTCAVHVRGGDIMGPAGAYFK HAMERMEQEKPGVRYIVFTNDMERAEEALAPVLESQKKDAVGQA ENRLEFVSEMGEFSDVDEFFLMAACQNQILSNSTFSTWAAYLNQNP DKTVIMPDDLLSERMRQKNWIILK | |

### Comparative example 1. Preparation of conventional α-1,2-fucosyltransferase

2FT derived from *helicobacter pylori* (FutC) was used for 2FL production in various documents due to the highest activity among 2FT enzymes, so it was selected as a control group.

In the Journal of Microbiol Res. 2019 May;222:35-42, entitled with Search for bacterial α1,2-fucosyltransferases for whole-cell biosynthesis of 2'-fucosyllactose in recombinant Escherichia coli, the reactivity of 2FT derived from *Thermosynechococcus elongates* was tested in *Escherichia coli,* and this was selected as a control group.

In disclosure of WO 2015/175801, the reactivity of 2FT derived from *Akkermansia muciniphila* was not confirmed, but was selected as a control group to re-exam the productivity.

A list and information of the selected enzymes of the control groups are shown in Table 3.

**[Table 3]**

| Name | Donor | accession number |
|---|---|---|
| Te2FT | *Thermosynechococcus elongatus* | WP_011056838.1 |
| Fut C (Hp2FT) | *helicobacter pylori* | WP_080473865.1 |
| Am2FT_1 | *Akkermansia muciniphila* | WP_081429121.1 |

### Example 2. Cloning of expression plasmid for Escherichia coli

In order to test the activity of the selected enzymes in Example 1 and the control enzymes of Comparative example 1 in *Escherichia coli,* cloning was carried out in an enzyme expression plasmid.

Specifically, in order to optimize codons of the enzyme sequences selected in Example 1 for *Escherichia coli,* the sequences were entered in a codon optimization program (http://genomes.urv.es/OPTIMIZER/), and then *Escherichia coli* was selected to perform guided random codon optimization search. The obtained enzyme sequences were synthesized, and synthetic genes were amplified by conducting PCR with the primers shown in Table 5 according to conditions. For the plasmid amplification of pET24ma, according to TAG or TAA of the stop codon sequence in the synthetic gene, TAG_PET24ma_F or TAA_PET24ma_F PCR primers were used. The information and name of primer sequence for each enzyme are shown in Table 4.

**[Table 4]**

| Name | Primer sequence (5'→ 3') | SEQ ID NO: |
|---|---|---|
| Te2FT_F | catatgATTATCGTTCATCTGTG | 31 |
| Te2FT_R | CTCGAGTCACAGAACAATCCACC | 32 |
| Bt2FT_F | catatgAACGAAATCCACGTGCT | 33 |
| Bt2FT_R | CTCGAGTCACTGGCCAGCCGGAATC | 34 |
| Bm2FT_F | catatgAAAATTGTTCAGATTTCT | 35 |
| Bm2FT_R | CTCGAGTCAGTAAACGATCCAACCT | 36 |
| Lg2FT_F | catatgCTGTACGTTGAAATGGACG | 37 |
| Lg2FT_R | CTCGAGTTACGCGTCGATCAGGATC | 38 |
| Ls2FT_F | catatgGAAGAGATTCACACCCTG | 39 |
| Ls2FT_R | CTCGAGCTAACGGCCGGCAGGTAC | 40 |
| L12FT_F | catatgTCTAAAAACATTTATGTAC | 41 |
| L12FT_R | CTCGAGTTAGATTTTGATCCAGTTGT | 42 |
| Am2FT_1_F | aagaaggagatatacatatgATGGCTGGACATTCTTGCG | 43 |
| Am2FT_1_R | tggtggtggtggtgctcgagTTAAATGCGCAGCCATGAGC | 44 |
| Am2FT_2_F | aagaaggagatatacatatgATGAACATGGAACGTAAGA | 45 |
| Am2FT_2_R | tggtggtggtggtgctcgagTTACAGGGTCACCCAGTGCG | 46 |
| Fp2FT_F | aagaaggagatatacatatgATGATTTACGTGGAAATG | 47 |
| Fp2FT_R | tggtggtggtggtgctcgagTTATTCGTTAACGATGTTA | 48 |
| Ri2FT_F | aagaaggagatatacatatgATGATCGCAGTTAAGATC | 49 |
| Ri2FT_R | tggtggtggtggtgctcgagTTATTTCAGGATAATCCAGT | 50 |
| Bm2FT_2_F | aagaaggagatatacatatgGGTTGCATTAAACGCCTGT | 51 |
| Bm2FT_2_R | TGGTGGTGGTGGTGCTCGAGCTATTCCAGGTGAGTGTTCA | 52 |
| Bm2FT_3_F | aagaaggagatatacatatgATCATTGTCCAGATTAAA | 53 |
| Bm2FT_3_R | TGGTGGTGGTGGTGCTCGAGCTATTGAAGATCCGCTTTGAT | 54 |
| Bm2FT_4_F | aagaaggagatatacatatgATCATTGTGCGTGTTATC | 55 |
| Bm2FT_4_R | TGGTGGTGGTGGTGCTCGAGTTAGAACGTATTAATACACT | 56 |
| Bs2FT_F | aagaaggagatatacatatgAAAATTGTAAAAGTTATC | 57 |
| Bs2FT_R | TGGTGGTGGTGGTGCTCGAGTTAGTAGATGATCCAGTCTT | 58 |
| Gs2FT_F | aagaaggagatatacatatgAAAATTGTAAAAGTTA | 59 |
| Gs2FT_R | TGGTGGTGGTGGTGCTCGAGTTAGATTTTAATCCATTCT | 60 |
| TAG_PET24 ma_F | TAGctcgagcaccaccaccaccacc | 61 |
| TAA_PET24 ma_F | TAActcgagcaccaccaccaccacc | 62 |
| PET24ma_R | Catatgtatatctccttcttaaagttaaacaaaattattt | 63 |

The synthetic genes of Te2FT, Bt2FT, Bm2FT, Lg2FT, Ls2FT, and L12FT were treated with 1uL NEB restriction enzyme (Nde1/ Xho1) respectively, after gene purification, and stored at 37°C for 3 hours. Then, in order to remove the restriction enzyme and buffer, the gene purification was carried out again, and ligation was conducted at 16°C for 4 hours by using pET24ma treated with the same restriction enzyme. All the remaining synthetic genes other than the synthetic genes of Te2FT, Bt2FT, Bm2FT, Lg2FT, Ls2FT, and Ll2FT were subjected to the gel prep to carry out cloning according to the Gibson assembly method.

After the gel prep, each gene concentration was measured by nano drop, and was added with 2X NEBuilder^{®} HiFi DNA Assembly Master Mix, so that the ratio of the vector and insert was 1:2 and the total reaction volume was 8uL. After that, it was reacted at 50°C for 1 hours. After transforming the cloned gene into a DH10b *E.coli* competent cell, it was smeared in a plate containing Km antibiotic and grown overnight.

Five (5) colonies grown in each plate were selected and subjected to colony PCR using the primers of Table 5 corresponding to the genes. The colony in which a band corresponding to the size on DNA gel was inoculated in a 3ml LB medium containing Km antibiotic, and then grown overnight. On the next day, cells were collected and DNA was extracted and sequenced.

### Example 3. Analysis of enzyme protein expression

The protein expression of each gene cloned in *Escherichia coli* expressed protein was analyzed. Each 2FT vector and pCDFm::PT7manC-manB and PT7-gmd-wcaG having an enzyme producing GDP-fuc to be experimented in the E.coli BL21(DE3) ΔfuclΔfucK::AprR lacZ△m15 ::PlacUV5 competent cell were transformed together. Selection was performed by smearing it on a plate containing Km and Amp antibiotics.

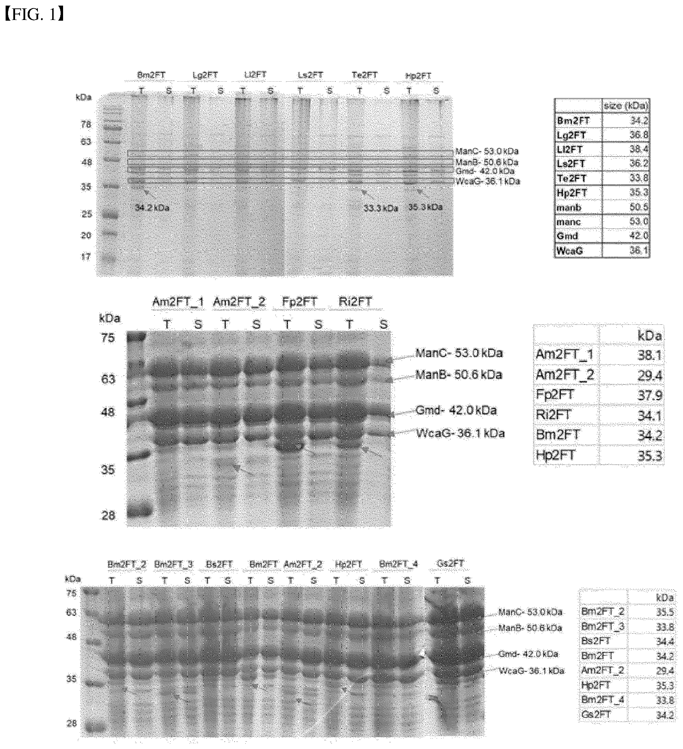

The colony was inoculated in a 3mL medium comprising Km and Amp antibiotics, and then seed culture were carried out overnight. After adding 200uL of seed culture solution and 0.1mM IPTG to 4mL LB, they were grown at 30°C for 16 hours. After all the cells grown in 4mL were collected with a centrifuge, the supernatant was discarded. After adding buffer of 500uL and then crushing with a bead beater, soluble proteins were collected by centrifuging at 13000 rpm for 20 minutes. They were run down SDS-PAGE gel with a size marker. The SDS-PAGE result of whole protein expression and soluble protein expression of the selected enzymes (left of FIG. 1) and the predicted protein size (right of FIG. 1) were shown in FIG. 1. In FIG. 1, ManB, ManC, Gmd, and WcaG are proteins expressed by additionally-added pCDFm::PT7manC-manB + PT7-gmd-wcaG plasmid.

As shown in FIG. 1, eight enzymes, Bm2FT, Te2FT, Hp2FT, Am2FT_2, Fp2FT, Ri2FT, Bm2FT_2, and Bm2FT_3 were confirmed their overexpression among the enzyme proteins compared through SDS-PAGE analysis.

### Example 4. Optimization of reaction analysis using test tube

The 2FL productivity was analyzed for 50 mL culture in a 250mL flask. In case of the large number of enzyme candidates, it is necessary to reproduce experiments 3 times or more for definite comparison analysis, but the conventional 50 mL culture condition has a disadvantage in that it requires more labor than that of the required amount(1mL). For rapid identification of reaction tendency of various candidate groups, a method for testing an enzyme reaction with 4mL culture in a 10mL test tube is to be established.

Plasmids cloned with Hp2FT, Bm2FT, and Bt2FT were transformed into the *E.coli* strain together with the pCDFm::PT7manC-manB + PT7-gmd-wcaG plasmid, respectively. Three colonies were inoculated in each plate, and seed culture was carried out overnight.

500uL among the seed was inducted in a 50mL flask culture. When the OD₆₀₀ reached about ~0.8, 0.1mM IPTG and 10mM lactose were added, and then it was analyzed by HPLC after 20 hours.

In case of the test tube reaction, seed 200 uL was added to 4mL medium added with 0.1mM IPTG and 10mM lactose. The test tube reaction could omit a step of measuring OD of the flask.

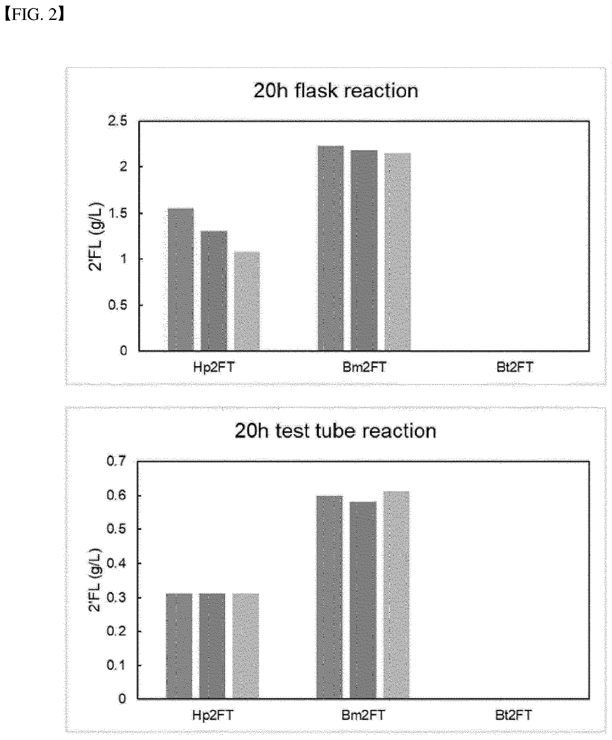

After 20 hours, the 2FL production tendency of the flask reaction (top of FIG. 2) and test tube reaction (bottom of FIG. 2) were analyzed with HPLC and shown in FIG. 2. As shown in FIG. 2, the 2FL productivity was at a level of 1/3 in the established test tube reaction condition compared to the flask condition, but the reaction tendency was same. Thus, it was confirmed that the test tube reaction was suitable for comparison analysis of various enzymes at a time.

### Example 5. Comparison of 2FL productivity through HPLC analysis

The reactivity of the enzymes selected in Example 1 and 3 kinds of the control enzymes were compared.

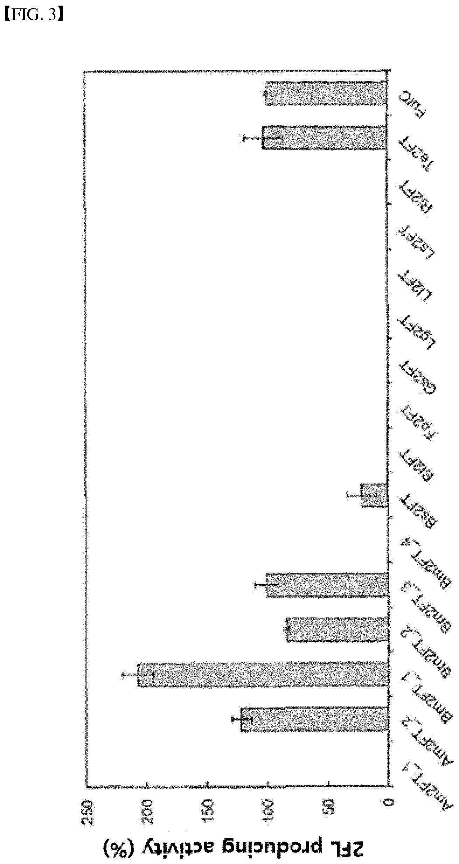

*E.coli* were transformed with plasmids to be compared respectively, and then grown at 37°C. Three colonies were inoculated in each plate, and overnight seed culture was conducted. Seed 200 uL was added to a 4mL medium added with 0.1mM IPTG and 10mM lactose. After 20 hours, it was analyzed by HPLC. The result of comparing the 2FL producing activity for the various 2FTs using the *E.coli* as a host was shown in FIG. 3.

As shown in FIG. 3, five enzymes producing 2FL were obtained, which included three enzymes being equal to or higher enzyme activity than FutC: Am2FT_2, Bm2FT, Bm2FT_2, Bm2FT_3, and Bs2FT respectively had 2FL production profile of 122%, 207%, 84%, 100%, or 21% aspects compared to FutC through HPLC analysis. The sequences of five enzymes producing 2FL are shown in Table 5.

**[Table 5]**

| Name | Sequence | SEQ ID NO: |
|---|---|---|
| Am2FT_2 | MNMERKTGLMNKKYVSPCFLPGMRLGNIMFTLAAACAHARTVGV ECRVPWAYNDASLMLRSRLGGWVLPSTPCGTNEPPSWQEPSFAYC PVPSRIRTGGLRGYFQSARYFEGQEAFIRALFAPLTAEKEPGAVGIHI RLGDYRRLRDKHRILDPGFLRRAAGHLSSGKNRLVLFSDEPDEAAE MLARVPAFGRFALEIDRGAPCESLRRMTAMEELVMSCSSFSWWGA WLGNTRKVIVPRDWFVGGVEDYRDIYLPHWVTL | 1 |
| Bm2FT | MKIVQISSGLGNQLFQYALYKRLSMNNNDVFLDVETSYQLNKNQH NGYEIERIFSIQPSHATKGMIDELADVDNRLINRLRRKLFGPKNSMY TETKEFSYDCEVFTKDGIYIKGYWQNYNYFKEIEDDLKNELVFKKA LDLKNSHLINQMNKEISVSIHVRRGDYYLNKEYENKFGNIADLDYY LKAINFIKKEVDDPKFYVFSDDIKWAKENLNLTDNVTYVEHNKGS DSYKDMRLMTCCKHNIIANSTFSWWGAFLNENKNKIVIAPGKWIN VEGVGGINLFPEGWIVY | 2 |
| Bm2FT_2 | MGCIKRLFLYEYGGRCFLVIVKIKGGFGNQLFTYASAYAISQELQQ NLIMDKVIYDLDYFRKFELPSLSLKYDQMLISKFVPNTKVKTLIYKV LRRLKLKGFTEVHEKKEFSFDENIYNLSGDIYLDGYWQNYRYFHK YYKDLSEMFVPRETPRKEVTDYITSLRGVNSVAMHVRRGDYKTFN GGKCLSLDYYIKAMEYFNSNDVQFYVFTDDIDFCEKNLPNSENINY VSRSEKLTDIEEFFIMKECKNFIIANSSFSWWAAYLSEQKADSLIVAP VVDMWKRDFYPDEWVALNTHLE | 3 |
| Bm2FT_3 | MIIVQIKGGLGNQLFSYASAYGIARENETELIIDRYIYDTSYSLRKYM | 4 |
| | LDFFPEIDEALLLKYIPKKNKISQIMYKLIRKSKLKYKYKAQLFLEEE EFKFTRISTQNENLYLNGYWQSYVYFDKYRNDIIKKFTPLVSFNQD GNQLLNEIKSYNSVAIHVRRGDYINFKGGKCLDSSYYIKAMKHLYN LKGKNLFFYIFTDDVEYCKRIFKNVANVKFIGEEAKLSDFEEFTLMT HCKNLIIGNSSFSWWAAYLASCKDKAVIAPVVDMWTEDFYLPEWI KIKADLQ | |
| Bs2FT | MKIVHISSGLGNQMFQYALYKKLSLIQDNVFLDTITSYQLYPNQHN GYELEKVFTIKPRHASKELTYNLSDLDNSVTSRIRRKLIGSKKSMYI EHKEFEYDPNLFYQENIYIKGYWQNYDYFKDIENELKNDFTFQRAL DEKNNNLAIKINNENSISIHVRRGDYYLNRKNQEKFGDIANLEYYS KAISYIKERIDNPKFYIFSDNVEWVKQNLNSLEEAVYIDYNVGNDSY KDMQLMSLCKHNIIANSSFSWWGAFLNKNMEKIVIAPGKWINMKG VKKVNLFPKDWIIY | 5 |

### Example 6. Comparison of 2'-FL productivity using fucosyltransferase in Corynebacterium strain

For the plasmid construction and 2'-fucosyllactose (2'-FL) production, *Corynebacterium glutamicum* (*C*. *glutamicum)* ATCC 13032 was used. In order to construct pMBC plasmid, manB gene was amplified through PCR reaction using two DNA primers from genomic DNA of *Corynebacterium glutamicum* ATCC 13032, and then was inserted into pCES208 plasmid. In the constructed plasmid, manC gene was amplified through PCR reaction using two DNA primers from genomic DNA of *Corynebacterium glutamicum* ATCC 13032 again, and was inserted into the plasmid, thereby constructing pMBC. In addition, in order to construct pEGW plasmid, gmd-wcaG gene cluster was amplified through PCR reaction using two DNA primers from genomic DNA of *E. coli* K-12, and then was inserted into the plasmid to construct pEGW. In addition, it was introduced by fucosyltransferase of SEQ ID NO: 2.

In case of *C.glutamicum* was used as a host, the strain introduced by Bm2FT and GDP-fucose cassette was added to a 4 mL medium added with 10 mM lactose, and cultured to produce 2FL. After 60 hours of culture, about 120 ng/L of 2FL was produced. The strain introduced by pMBC and pEGW empty vectors did not produce 2FL.

### Example 8. Comparison of 2'-FL productivity through fucosyltransferase in Bacillus megaterium strain

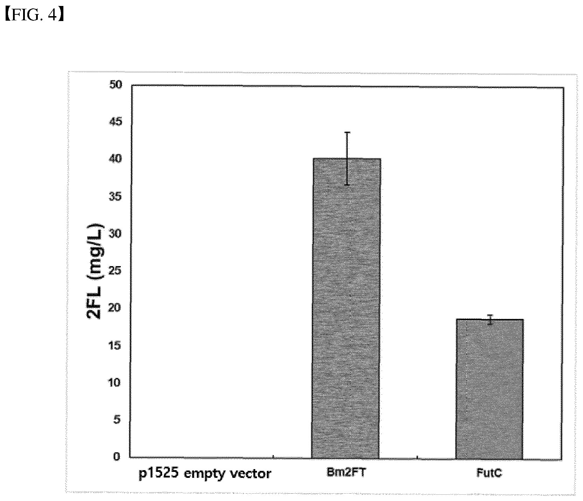

The Am2FT_2, Bm2FT enzymes in which their activity were most increased compared to FutC in *E.coli* as a host were transformed into the *B. megaterium* 14581 (Korean Culture Center of Microorganisms (KCCM) accession number 40441) as a host respectively, to compare their 2FL productivity. For accurate analysis of the reactivity, *B. megaterium* 14581 introduced by the empty vector p1525 containing no 2FT gene was used as a control group. FIG. 4 is a drawing which shows the result of comparing various 2FL productivity using the *B.megaterium* 14581 as a host.

As shown in FIG. 4, in case of *B. megaterium* host, the strain inserted by the p1525 empty vector did not produce 2FL, but the strain inserted by Bm2FT and FutC plasmid produced 40.3 mg/L and 18.8 mg/L of 2FL on average, respectively after 64 hours of culture. Such result confirmed that the Bm2FT enzyme showed an increase in the productivity by 2.1 times or more compared to FutC.

No research on production of human milk oligosaccharides using *B. megaterium* strain has been reported so far, and *B. megaterium* 14581 host is harmless to a human body due to Biosafety level 1 strain. Therefore, the productivity by 2 times or more compared to the conventional enzyme (FucT) was obtained by introducing Bm2FT of the present invention into the GRAS strain.

### Example 9. Comparison of 2'-FL productivity through fucosyltransferase in Bacillus subtilis strain

The Bm2FT enzymes in which the activity was increased the most compared to FutC in *E.coli* host were transformed into *Bacillus subtilis* as a host to compare 2'-FL productivity. For accurate analysis of the reactivity, *B. subtilis* 168 (accession number: ATCC^{®} 23857^{™}) transformed with an empty vector pBE-S in which the 2'-FL gene was not cloned was used as a control group. The used vector was pBE-S for *B.subtilis* secretion, and aprE promoter capable of constitutive expression was contained. As the secretory function is not required during cloning, aprE signal peptide was removed when performing 2'-FL cloning.

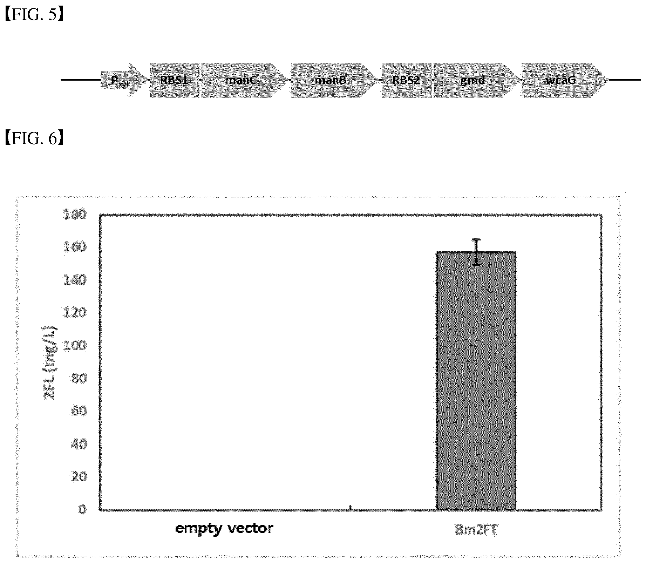

As *B.subtilis* 168 does not have 4 kinds of enzymes producing GDP-fucose, genes encoding the 4 kinds of enzymes were cloned into one vector at a time. The 4 kinds of genes (manB, manC, gmd, wcaG) were derived from *E. coli* and obtained by gene amplification of PCR. A vector used for cloning the 4 kinds of genes at a time was p3STOP1623-2RBShp, which was an *E.coli-Bacillus* shuttle vector. The vector could be used in not only *B.megaterium,* but also *B.subtilis.* As a characteristic of the vector, it had two RBSs, so it is very easy for cloning of multiple genes. The promoter of the vector is PxylA, which is a xylose inducible promoter. The GDP-fucose cassette was constructed in the gene order as FIG. 5.

The vector and 4 kinds of genes were cloned by applying Gibson assembly method as same as Bm2FT cloning as described above. However, as the vector size was about 6.6 Kb, sequence errors might be generated to perform PCR together, so PCR was carried out for the divided half region by constructing primers for the middle region of the vector. In order to purify all six PCR fragments, all PCR mixtures were electrophoresed and subjected to gel extraction. The PCR fragments obtained by purification were analyzed for measuring the DNA concentration, and Gibson assembly was conducted by adjusting the content according to the concentration and size. After that, a single colony was obtained by transforming it into *E.coli* in the same way, and then, the insertion of the plasmid was tested by performing the colony PCR. PCR-confirmed colonies were cultured in LB medium, and plasmids were extracted. Then, it was confirmed that 4 kinds of genes were properly cloned through sequence analysis. The plasmid of which sequence was confirmed was transformed into *B.subtilis* 168 for productivity analysis. After confirming insertion of the plasmid by colony PCR as same test method of *E.coli* transformation, culture was performed to compare 2'-FL productivity.

As shown in FIG. 6, using the *B.subtilis* host, the strain in which pBE-S and p3STOP1623-2RBShp empty vectors were inserted did not produce 2FL, and the strain in which the Bm2FT and GDP-fucose cassette were introduced produced about 150 mg/L of 2FL after 63 hours of reacting. This is the result that the productivity is improved by about 7 times or more compared to the average 2FL production amount of 18.8 mg/L of the conventional FutC .

### Example 10. Comparison of 2'-FL productivity through fucosyltransferase in Saccharomyces Cerevisiae strain

For construction of plasmids and production of 2'-fucosyllactose (2'-FL), *Saccharomyces cerevisiae (S. cerevisiae)* CEN.PK2-1c was used. A GDP-L-Fucose gene not present in *Saccharomyces cerevisiae* was cloned from *E.coli* K-12 and used. In order to construct pRSK426 plasmid, after amplifying gmd-wcaG gene cluster through PCR reaction, it was inserted into the plasmid to construct pRSK426. The Am2FT_2, Bm2FT enzymes in which the activity was most increased compared to FutC when using the *E.coli* host were respectively transformed into the S. cerevisiae host to compare each 2FL productivity. The 3 kinds of recombinant plasmids were transformed into the *Saccharomyces cerevisiae (S. cerevisiae)* CEN.PK2-1c strain and then selected in YPD solid medium comprising G418 antibiotics. The selected strains were cultured in a 50mL liquid production medium comprising G418 antibiotics and then the 2FL productivity was measured by HPLC analysis. Using the *S*. *cerevisiae* CEN.PK2-1c host, the strain in which the pRSK426 empty vector was inserted did not produce 2FL, and the strain in which the Bm2FT, Am2FT_2 and GDP-fucose cassette were inserted produced 2FL of 80 mg/L, 40 mg/L, respectively, after 48 hours of culture.

## Claims

1. A recombinant microorganism, transformed to express α-1,2-fucosyltransferase comprising at least one amino acid sequence selected from the group consisting of SEQ ID NOs: 1 to 5.

2. The recombinant microorganism according to claim 1, wherein the recombinant microorganism is a GRAS (Generally Recognized As Safe) microorganism.

3. The recombinant microorganism according to claim 1, wherein the recombinant microorganism is *Bacillus* sp. microorganism, *Corynebacterium* sp. microorganism, *Escherichia* sp. microorganism, or yeast.

4. The recombinant microorganism according to claim 3, wherein the *Bacillus* sp. microorganism is *Bacillus megaterium, Bacillus subtilis, Bacillus cereus, Bacillus coagulans, Bacillus licheniformis,* or *Bacillus stearothermophilus,*
the *Corynebacterium* sp. microorganism is *Corynebacterium glutamicum,*
the *Escherichia* sp. microorganism is *Escherichia coli,* and
the yeast is *Saccharomyces cerevisiae,* or *Candida utilis.*

5. The recombinant microorganism according to claim 1, wherein the microorganism does not have 2'-fucosyllactose productivity before recombination, and obtain 2'-fucosyllactose productivity by recombination.

6. The recombinant microorganism according to claim 1, wherein the recombinant microorganism expresses a fucose synthesis gene.

7. The recombinant microorganism according to claim 6, wherein the fucose synthesis gene expresses at least one selected from the group consisting of GDP-D-mannose-4,6-dehydratase, GDP-L-fucose synthase, phosphomannomutase, and GTP-mannose-1-phosphate guanylyltransferase.

8. The recombinant microorganism according to claim 1, wherein the recombinant microorganism expresses a lactose membrane transport protein.

9. The recombinant microorganism according to claim 8, wherein the lactose membrane transport protein is at least one selected from the group consisting of Lac12 and LacY.

10. The recombinant microorganism according to claim 1, wherein the recombinant microorganism has 2'-fucosyllactose productivity of 2-fold or higher compared to a control group comprising α-1,2-fucosyltransferase derived from *Helicobacter pylori.*

11. A method for producing 2'-fucosyllactose, comprising a step of culturing the recombinant microorganism according to any one of claim 1 to claim 10 in a medium comprising lactose.
